# FASCICULE DE BREVET EUROPEEN

(11) **EP 1 588 158 B1**
(45) Date de publication et mention de la délivrance du brevet: **07.03.2007**
(21) Numéro de dépôt: 03815377.1
(22) Date de dépôt: 30.12.2003
(51) Int. Cl.: G01N 33/03, G01N 27/22, A47J 37/12

(54) **MESURE DE LA QUALITE ET/OU DE LA DEGRADATION D'UNE HUILE ALIMENTAIRE, APPAREIL DE CUISSON**
MESSUNG DER QUALITÄT UND/ODER DER ZERSETZUNG VON SPEISEÖL, KOCHGERÄT
MEASURING THE QUALITY AND/OR DEGRADATION OF FOOD OIL, COOKING APPLIANCE

(30) Priorité: 20.01.2003 EP 03075203
(43) Date de publication de la demande: 26.10.2005
(73) Titulaire: Ecole Polytechnique Fédérale de Lausanne (EPFL), 1015 Lausanne (CH); Chambon, Gérald, 1018 Lausanne (CH)
(72) Inventeur: CHAMBON, Gérald, CH-1018 Lausanne (CH); GIJS, Martin, CH-1024 Ecublens (CH)
(74) Mandataire: Ravenel, Thierry Gérard Louis
(86) Numéro de dépôt international: PCT/EP2003/014955
(87) Numéro de publication internationale: WO 2004/065957

(56) Documents cités:
- WO-A-00/62057
- DE-A- 19 649 366
- GB-A- 2 136 130
- US-A- 4 728 882
- US-A- 4 733 556
- US-A- 5 111 221
- US-A- 5 818 731
- US-B1- 6 469 521

## Description

La présente invention concerne l'utilisation d'un dispositif de mesure capacitive de la qualité et/ou de la dégradation d'un fluide pour la mesure de la qualité et/ou la dégradation d'une huile alimentaire. L'invention concerne en particulier un tel dispositif permettant la mesure de la qualité et/ou de la dégradation d'huile alimentaire, telle qu'une huile de friture, directement dans l'appareil de cuisson.

Il est bien connu que les huiles alimentaires se dégradent lors de la cuisson, notamment lorsqu'elles sont portées de manière répétée à des températures élevées. Typiquement, pour frire des aliments ces huiles sont portées à des températures de l'ordre de 180°C. A ces températures se produisent une multitude de réactions chimiques, telles que des polymérisations, des thermo-oxydations, etc., qui altèrent de manière importante la qualité de l'huile. La quantité de certains produits des ces réactions ne doit pas dépasser un seuil imposé par la législation, car au-delà de ce seuil l'huile est considérée comme impropre à la consommation. Il est donc important de pouvoir détecter ce seuil de manière fiable afin de remplacer l'huile dès que cela est nécessaire. Pendant longtemps ce moment a été laissé à l'appréciation des cuisiniers qui suite à une inspection visuelle et/ou olfactive déterminaient si l'huile était encore propre à la consommation. Bien entendu un telle méthode est purement subjective et n'est par conséquent pas fiable.

Divers dispositifs ont été proposés dans l'art antérieur jusqu'ici pour tenter de remédier à ce problème afin de pouvoir mesurer de manière objective la qualité et/ou la dégradation d'huiles alimentaires. Comme la dégradation des huiles alimentaires résulte notamment de leur thermo-oxydation et que cette réaction produit des composés polaires, il a été envisagé des dispositifs dans lesquels on corrèle le degré de dégradation des huiles à la constante diélectrique de l'huile, en mesurant la capacité d'un condensateur dans lequel l'huile à surveiller forme le diélectrique.

Un tel dispositif est par exemple décrit dans le brevet US No 5 818 731. Ce document, décrit un dispositif de mesure de la qualité d'huiles alimentaires destiné à être monté dans un appareil de cuisson telle qu'une friteuse. Ce dispositif surveille simultanément les variations de capacité et de transmission optique de l'huile dans la gamme de température de cuisson ou de friture. L'unité de mesure capacitive comprend deux jeux de plaques parallèles imbriqués fun dans l'autre pour définir un condensateur de mesure. Lorsque les jeux de plaques sont immergés dans l'huile, cette dernière forme le diélectrique du condensateur de mesure de ladite unité et la variation de la capacité est mesurée au moyen d'un circuit en pont à oscillateur à courant continu. Ce dispositif présente toutefois divers inconvénients. Un premier inconvénient réside dans le fait que les espaces entre les plaques sont petits et que, lorsque les plaques sont plongées dans l'huile, cette dernière ne circule pas aisément entre les plaques en raison des phénomènes de capillarité. Le renouvellement régulier de l'huile présente entre ces plaques n'est donc pas garanti, ce qui peut conduire à des résultats erronés de mesure de la dégradation de l'huile. De plus, des particules solides présentes dans l'huile peuvent également se trouvées piégées entre les plaques, ce qui affecte négativement le signal mesuré. A noter également que la configuration en plaques parallèles du condensateur ne permet pas, compte tenu de du faible espace entre les plaques, un accès aisé à ces espaces ce qui rend compliquée les opérations de maintenance du dispositif. Un autre inconvénient réside dans le fait que le condensateur à plaques est encombrant et occupe une place importante dans l'appareil de cuisson. En outre, l'unique capteur de mesure formé par le condensateur est sujet aux variations de température ce qui peut conduire à des mesures erronées de capacité, de sorte que ce dispositif doit prévoir des moyens pour compenser ces erreurs. La solution proposée dans ce document consiste à utiliser un capteur de température qui fournit une indication à un circuit de traitement à même de prendre en compte les variations de température mesurées par des moyens logiciels intégrant des données relatives à l'huile à surveiller. Par conséquent, si la qualité de l'huile change ou si une nouvelle huile est utilisée, il est nécessaire de mettre à jour les moyens à logiciels ce qui rend le dispositif peu souple d'utilisation.

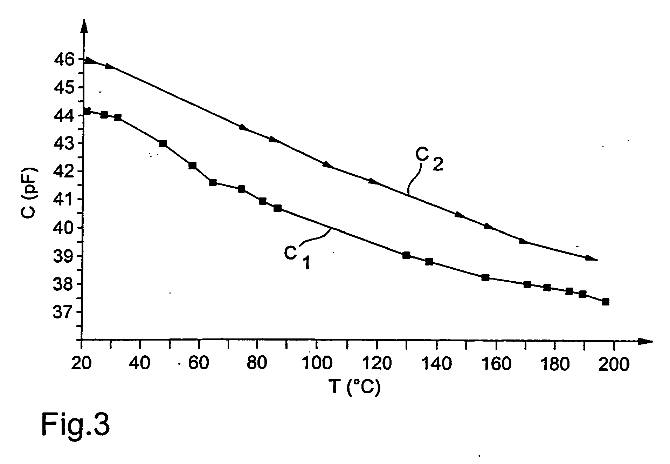

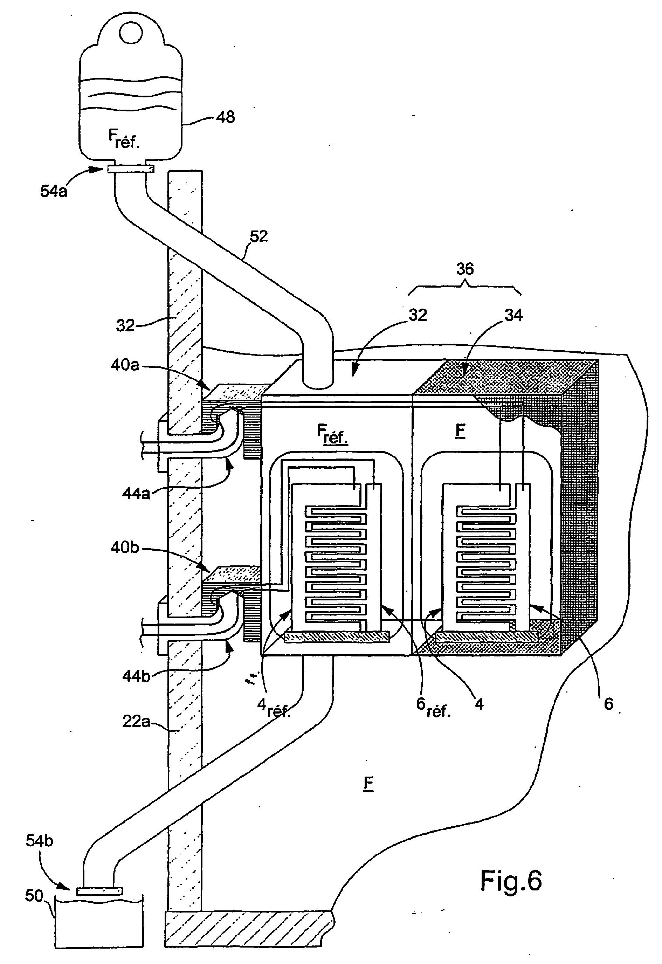

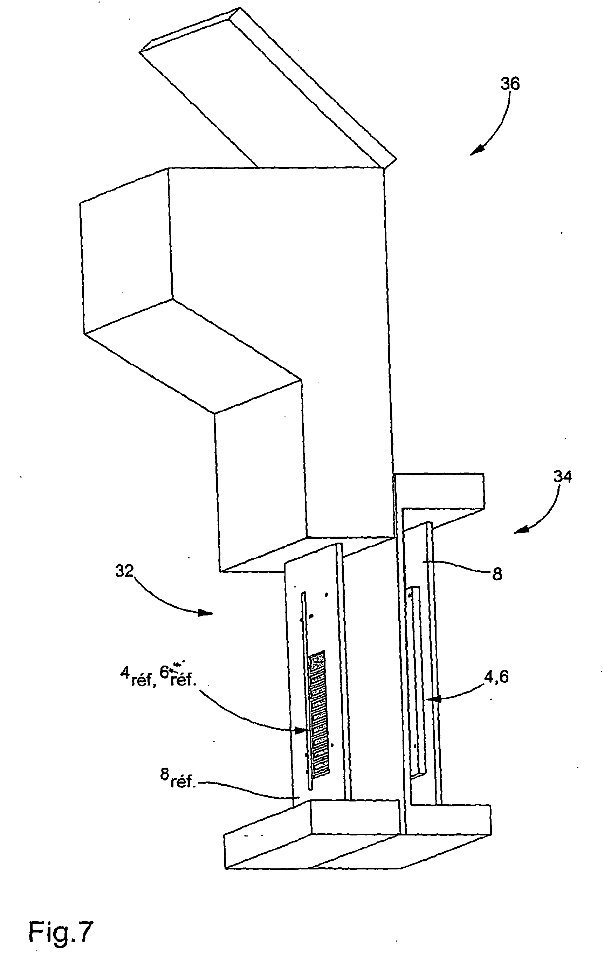

US-A-4 728 882 décrit un dispositif de mesure de la qualité d'un fluide comprenant un capteur mesurant la capacité du fluide, les électrodes du dit capteur se trouvant dans un même plan (voir Fig.3, 6), des moyens de traitement du signal et de présentation du résultat (voir Fig. 9, col. 9, 1.49-53 et revendication 21) ainsi qu'un capteur de référence (42), voir Fig. 7 et 9. Ce dispositif est adapté aux liquides contenant des hydrocarbures ou d'autres liquides (voir col. 8, 1.18-28). Néanmoins, le fluide ne baigne pas les deux faces des électrodes, "de part et d'autre dudit plan". La figure 7 concerne une électrode de mesure et une électrode de référence fixées dos à dos sur un support 52.

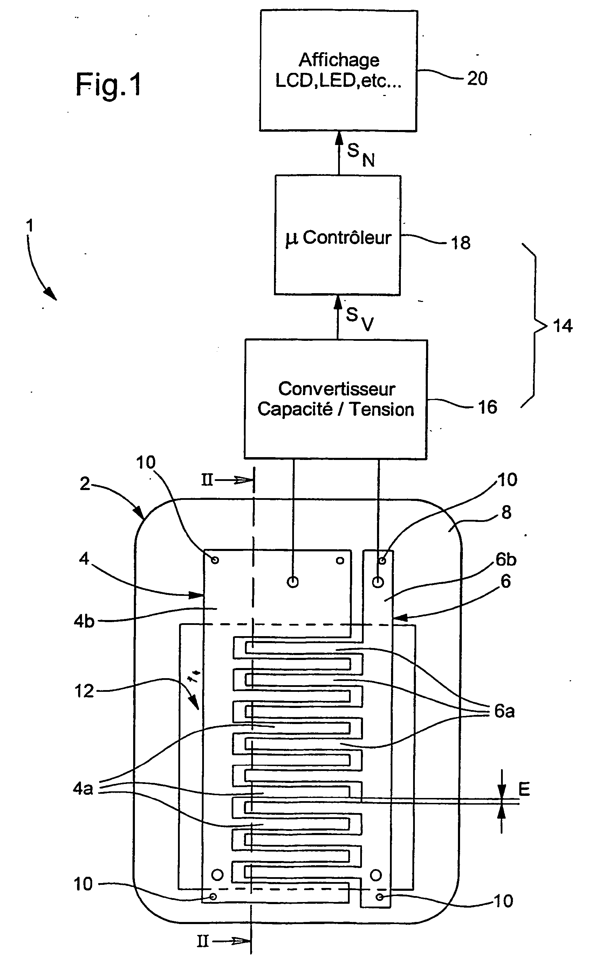

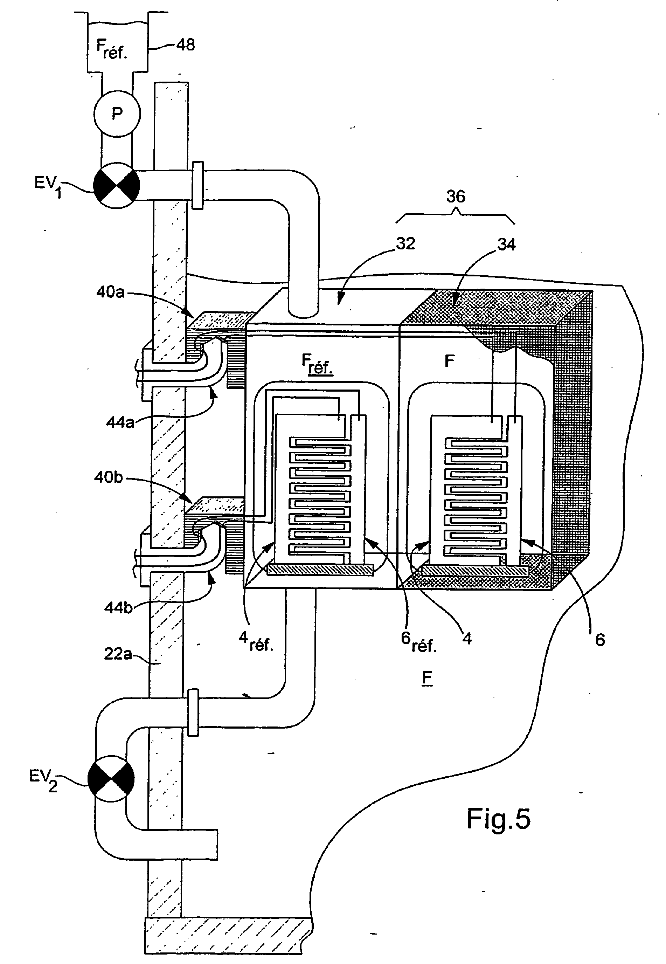

GB-A-2 136 130 décrit un dispositif adapté à la mesure de la qualité d'un fluide. Ce dispositif comprend des électrodes en forme de peigne, situés dans un même plan, agencées de façon à ce que le fluide baigne les deux faces de chaque électrode (voir Fig. 1, 2, 4, 5). Il comprend également une paire d'électrodes de référence, qui peut baigner dans un autre fluide (voir p.3, colonne 2, 1.85-97 et p.7, colonne 1, I.1-5). Les électrodes sont recouvertes d'un film réactif 26 ou 226 et placées sur un support 22 ou 222. Dans le mode de réalisation de la Fig. 5, ce support peut laisser circuler le fluide au sein de l'électrode (voir p. 7, colonne 1, 1.23-30). Le film diélectrique 24 ou 224 est une caractéristique courante dans l'état de la technique. D'après le passage p.7, colonne 1, 1.58-59, elle est néanmoins optionnelle. Dans ce dispositif, le fluide peut venir circuler "au voisinage des dents des électrodes".

DE-A-19649366 décrit un capteur pour mesurer la qualité d'un fluide (par exemple un mélange essence-alcool, colonne 1, 1.10-11). Ce dispositif comprend des électrodes en forme de peigne, situées dans un même plan, agencées de façon à ce que le fluide baigne les deux faces de chaque électrode (voir résumé, revendications, colonne 2, 1.32-36, colonne 4,1.12-16 et 1.55-58). Ce capteur peut aussi être utilisé pour la mesure de la qualité/dégradation d'huile de moteur (cf. colonne 6, 1.48-50).

US-B1-6 469 521 décrit un appareil de mesure portatif servant à mesurer la qualité d'une huile alimentaire ou de moteur. Il comprend des électrodes interdigitées situées dans un même plan et placées sur un support (voir Fig. 1A). Le fluide à mesurer ne peut pas traverser le plan des électrodes.

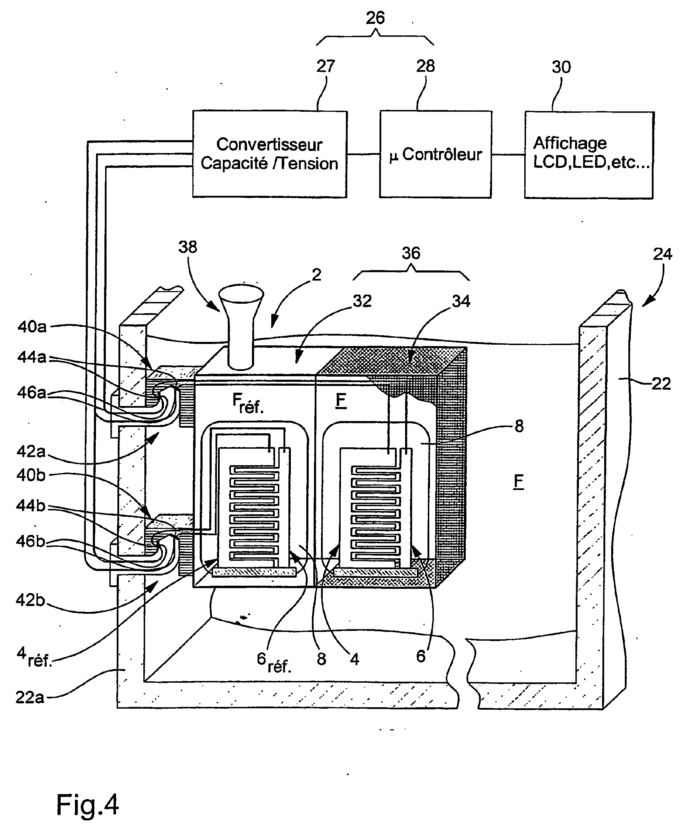

WO/00 62057 décrit un appareil de cuisson comprenant un jeu d'électrodes servant à mesurer la qualité d'une huile alimentaire. Ces électrodes ne sont pas situées dans un même plan (Voir Fig. 4).

La présente invention a pour but principal de remédier aux inconvénients de l'art antérieur susmentionné en utilisant un dispositif amélioré de mesure de la qualité et/ou de la dégradation d'un fluide par mesure capacitive, présentant une construction simple, compacte et peu coûteuse.

La présente invention a également pour but l'utilisation d'un tel dispositif dans lequel le capteur de mesure capacitif présente une structure permettant une circulation facilitée du fluide à mesurer au voisinage de ses électrodes tout en conservant une sensibilité élevée de mesure de variation de la capacité.

La présente invention a également pour but l'utilisation d'un tel dispositif dans lequel la probabilité que des particules présentes dans le fluide se trouvent piégées entre les électrodes du condensateur de mesure est réduite.

La présente invention a également pour but l'utilisation d'un tel dispositif dont l'entretien, et notamment le nettoyage du capteur de mesure est facilité.

La présente invention a également pour but l'utilisation d'un tel dispositif permettant de s'affranchir de la dépendance en température de la mesure de la capacité tout en conservant une grande souplesse d'utilisation, c'est-à-dire un dispositif qui ne nécessite pas une mise à jour systématique des moyens logiciels du circuit de traitement dès que l'on souhaite utiliser le dispositif avec un fluide de nature différente.

A cet effet, l'invention a pour objet l'utilisation d'un dispositif de mesure de la qualité et/ou de la dégradation d'un fluide, notamment d'une huile, comprenant un capteur comportant au moins une paire d'électrodes espacées l'une de l'autre, destiné à être immergé dans le fluide à mesurer, les électrodes et le fluide formant un élément capacitif de mesure dont la capacité varie en fonction de la constante diélectrique du fluide. Le capteur est capable de fournir un signal électrique de sortie représentatif de ladite constante diélectrique. Le dispositif comprend en outre des moyens de traitement recevant ledit signal de sortie et capables de déterminer le degré de qualité et/ou de dégrada6on du fluide sur la base dudit signal de sortie. Ce dispositif est caractérisé en ce que lesdites électrodes s'étendent sensiblement dans un même plan et en ce que le fluide baigne les deux faces des électrodes, de part et d'autre dudit plan.

Grâce à ces caractéristiques, le fluide à mesurer peut circuler aisément et rapidement de part et d'autre des électrodes de l'élément capacitif de mesure. Le fluide présent au voisinage des électrodes peut être ainsi constamment renouvelé, ce qui améliore la fiabilité et la précision des mesures fournies par le dispositif dans la mesure où celles-ci sont représentatives de l'évolution de la qualité du fluide dans son ensemble. Cette structure permet par ailleurs de diminuer fortement le risque que des particules se coincent dans l'entrefer de l'élément capacitif. Un autre avantage du dispositif de l'invention réside dans le fait que l'accès à l'entrefer est aisé, ce qui facilite les opérations d'entretien du capteur. A noter également que comme le fluide baigne les faces des électrodes de mesure de part et d'autre de leur plan médian, il est possible d'obtenir une grande sensibilité de mesure.

Selon un mode de réalisation préféré de l'invention, le capteur comprend en outre un élément capacitif de référence, comportant au moins une paire d'électrodes de références espacées l'une de l'autre, destiné à être immergé dans un fluide de référence, les électrodes et le fluide de référence formant un élément capacitif de référence dont la capacité varie en fonction de la constante diélectrique du fluide de référence, ledit élément capacitif de référence étant capable de fournir un signal de référence représentatif de ladite constante diélectrique de référence aux moyens de traitement et les moyens de traitement étant agencés pour comparer le signal de sortie avec le signal de référence.

L'élément capacitif de référence peut ainsi mesurer en permanence les propriétés diélectriques d'un fluide de référence « neuf » ou en d'autres termes non dégradé, et fournir une valeur de constante diélectrique de référence de ce fluide qui peut être comparé à la valeur de constante diélectrique fournie par l'élément capacitif de mesure. L'utilisation de deux capteurs permet également de s'affranchir des variations de la constante diélectrique dues aux variations de la température.

lorsque le dispositif de mesure est associé à un appareil de cuisson comprenant une cuve contenant de l'huile alimentaire de cuisson, l'élément capacitif de mesure peut baigner dans l'huile dans laquelle les aliments sont frits tandis que l'élément capacitif de référence baigne dans une huile alimentaire de référence ayant les mêmes caractéristiques que l'huile alimentaire de cuisson, mais contenue dans une enceinte isolée de celle-ci.

De préférence, l'enceinte contenant l'huile alimentaire de référence en contact thermique avec l'huile alimentaire de cuisson. L'huile alimentaire de référence peut bien entendu être renouvelée périodiquement, par exemple une fois par jour ou, le cas échéant, de manière continue pour fournir une valeur de constante diélectrique de référence bien définie pour une huile non dégradée. Ce renouvellement peut être réalisé de manière automatique ou manuel.

D'autres caractéristiques et avantages de la présente invention apparaîtront dans la description suivante de modes de réalisation préférés de dispositifs de mesure présentés à titre d'exemples non limitatifs en référence aux dessins annexés, dans lesquels :
- La figure 1 est une vue schématique d'un premier mode de réalisation du dispositif de mesure ;
- La figure 2 est une vue schématique en coupe d'une cuve d'un appareil de cuisson classique sur laquelle est fixé le dispositif de mesure représenté à la figure 1, ce dernier étant partiellement représenté en coupe selon la ligne 11-11 de la figure 1 et les moyens de traitement ayant été omis ;
- La figure 3 montre un graphe représentant, en fonction de la température, la variation de la capacité d'un élément capacitif du dispositif de mesure lorsque l'élément capacitif baigne dans une huile neuve et dans une huile usagée ;
- La figure 4 est une vue schématique en coupe et en perspective d'une cuve d'un appareil de cuisson classique sur laquelle est fixé un dispositif de mesure selon un deuxième mode de réalisation ;
- La figure 5 représente une variante du deuxième mode de réalisation du dispositif de mesure
- La figure 6 représente une variante du mode de réalisation illustré à la figure 5. ; et
- La figure 7 représente un exemple de réalisation d'une structure de support des éléments capacitifs du dispositif ;

En se référant tout d'abord à la figure 1, on voit un premier mode de réalisation d'un dispositif de mesure capacitive de la qualité et/ou de la dégradation d'un fluide notamment d'une huile, désigné par la référence générale 1.

On notera que la description qui suit sera faite dans une application du dispositif 1 à la mesure de la qualité et/ou de la dégradation d'une huile alimentaire ou analogue, utilisée pour frire des aliments dans des appareils de cuisson comprenant une cuve dans laquelle l'huile peut être chauffée typiquement jusqu'à environ 200°C.

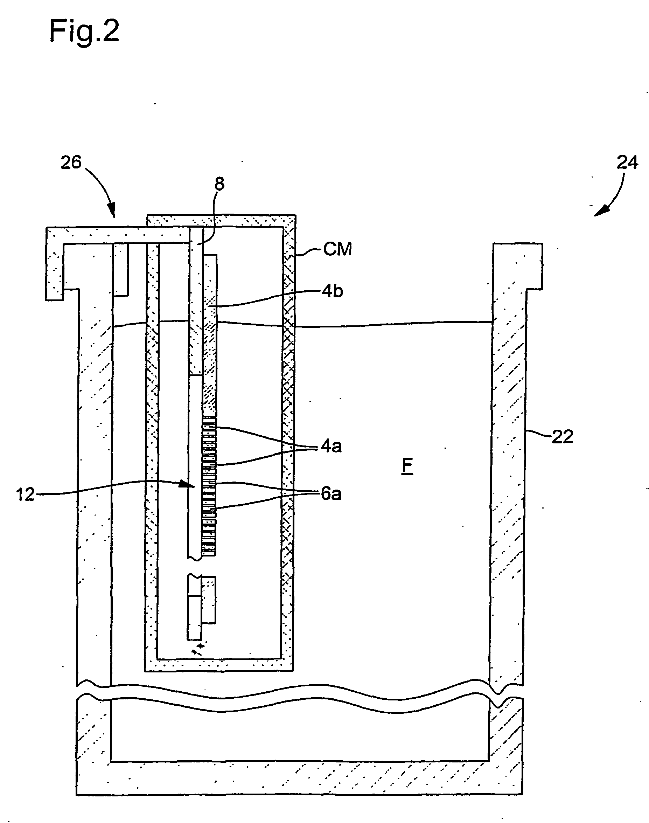

Le dispositif de mesure 1 comprend un capteur 2 comportant une paire d'électrodes 4, 6 espacées l'une de l'autre et destinées à être immergées dans un fluide F (Figure 2), par exemple l'huile d'une friteuse, dont on souhaite mesurer la qualité et/ou la dégradation et déterminer si elle est toujours propre à l'emploi. Les électrodes 4, 6 forment avec l'huile F un élément capacitif de mesure EFM dont la capacité varie en fonction de la constante diélectrique de l'huile. Lorsque l'huile se dégrade, la quantité de composés polaires présents dans celle-ci augmente et conduit à une augmentation de sa constante diélectrique. Ainsi, en mesurant l'évolution de la capacité de l'élément capacitif de mesure EFM, on peut déterminer le degré de qualité et/ou de dégradation de l'huile. Le capteur 2, et plus précisément son élément capacitif EFM, est donc capable de fournir un signal électrique de sortie représentatif de la constante diélectrique de l'huile sur une large plage de températures, en particulier entre 20°C et 200°C.

Chaque électrode 4, 6 de la paire présente la forme d'un peigne ayant une pluralité de dents 4a, 6a sensiblement parallèles entre elles et s'étendant à partir d'une base 4b, 6b. Les électrodes 4 et 6 sont disposées l'une par rapport à l'autre de sorte que les dents 4a d'une électrode 4 sont imbriquées entre les dents 6a de l'autre électrode 6. Les dents des électrodes 4 et 6 sont ainsi disposées sensiblement dans un même plan.

On notera à ce propos que les électrodes 4 et 6 sont par exemple formées à partir d'une même plaque plane découpée de façon appropriée, la plaque étant suffisamment rigide pour que les électrodes gardent leur forme lorsqu'elles sont manipulées. Dans l'exemple décrit, les électrodes sont réalisées à partir d'une plaque en un acier alimentaire (acier inox austénitique de type 18-10 à bas carbone) ayant une épaisseur comprise entre 0,1 et 3 mm. D'autres types d'aciers alimentaires peuvent être également utilisés par exemple le Z7CN18-09, le Z3CND18-12-02, Z6CNDT17-12 et Z7CNU16-04. La plaque est découpée au moyen d'un faisceau laser, ce qui permet de réaliser entre les dents des électrodes des entrefers compris entre 10 nm et 1 mm. Il est bien entendu que plus l'entrefer E est faible, plus la sensibilité de l'élément capacitif est grande. Selon une variante de réalisation, on peut également envisager de réalisé des électrodes formées d'un substrat enrobé d'un matériau conducteur, par exemple un substrat enrobé d'une couche d'or de platine ou analogue.

Dans le mode de réalisation représenté, les électrodes 4 et 6 sont solidaires d'un substrat 8 isolant qui maintient, en liaison avec des moyens d'alignement 10, les électrodes dans une position fixe l'une par rapport à l'autre. Plus précisément, les électrodes 4 et 6 sont chacune fixées au substrat 8 par l'intermédiaire d'une patte de fixation s'étendant à partir de leur base 4b, 6b par tout moyen approprié, par exemple par des vis ou analogues. Les moyens d'alignement 10 comprennent par exemple des goupilles de positionnement chassées dans le substrat 8 et coopérant avec des trous à cet effet dans les électrodes 4 et 6.

Le substrat 8 présente la forme d'un cadre ayant une ouverture centrale 12 disposée en regard de la région de mesure des électrodes 4 et 6, c'est-à-dire en regard des entrefers définis par les espaces entre les dents 4a de l'électrode 4 et les dents 6a de l'électrode 6. Grâce à cette configuration, le fluide à mesurer, en l'occurrence l'huile, baigne les deux faces des électrodes 4 et 6 de part et d'autre du plan de ces électrodes de sorte qu'elle peut venir circuler au voisinage des dents 4a et 6a des électrodes 4 et 6.

On notera que l'élément capacitif EFM est entouré d'un cadre métallique CM. Ce cadre métallique forme un écran protecteur vis-à-vis de perturbations électriques externes et permet de réduire ainsi l'influence de ces perturbations au cours des mesures. Ce cadre est typiquement formé par un grillage métallique.

Le substrat 8 est réalisé de préférence en un matériau résistant à des températures comprises entre 20°C et 200°C et présentant un faible coefficient de dilatation thermique, tel qu'un matériau céramique. Cependant il peut être réalisé en tout autre matériau isolant compatible avec l'application du dispositif de mesure envisagée. A titre d'exemple pour une application alimentaire devant être stable dans la gamme de température susmentionnée, le substrat 8 pourrait également réalisé en un polymère fluoré tel que le Téflon.

Pour fixer les idées, la demanderesse a effectué des essais concluant avec des électrodes 4, 6 en acier inoxydables présentant une épaisseur de l'ordre de 0,8mm. L'électrodes 4 comprenait 9 dents et l'électrode 6 comprenait 8 dents définissant 18 entrefers de 100 µm chacun, les dents présentant une largeur de l'ordre de 1 mm. Le substrat étant réalisé en céramique et présentait une épaisseur de l'ordre de 0,6 mm pour une surface extérieure 5x5 cm.

La capacité de l'élément capacitif de mesure EFM défini par les électrodes 4 et 6 en combinaison avec l'huile est mesurée par des moyens de traitement 14 comprenant par exemple un circuit analogique convertisseur capacité/tension 16 bien connu de l'homme de métier associé à un microcontrôleur 18. A titre d'exemple, le circuit convertisseur capacité/tension portant la référence XE2004 commercialisé par la société Xemics peut être utilisé.

Les électrodes 4 et 6 sont reliées en entrée du circuit 16 qui délivre en sortie un signal analogique de tension S_{V} représentatif de la capacité de l'élément capacitif de mesure. Le signal S_{V} est fourni en entrée du microcontrôleur 18 qui convertit le signal Sv en un signal numérique S_{N}. On pourra typiquement utiliser un microcontrôleur portant la référence 68HC11 et commercialisé par la société Motorola. Le signal numérique en sortie du microcontrôleur est ensuite fourni à des moyens d'affichage 20, réalisés par exemple sous la forme d'une cellule à cristaux liquides ou encore par un affichage à LED. Cette dernière affiche une valeur numérique représentative par exemple de la constante diélectrique de l'huile. Selon une variante de réalisation, cette valeur numérique peut être traitée de façon appropriée pour indiquer le taux de composés polaires mesuré dans l'huile.

En se référant à la figure 2, on voit comment l'élément capacitif de mesure EFM du dispositif de mesure 1 est disposé dans une cuve 22 d'un appareil de cuisson classique 24 contenant l'huile alimentaire à examiner. Les moyens de traitement 14 et d'affichage 20 sont omis dans cette figure. Ces derniers seront par exemple disposés dans un boîtier associé à l'appareil de cuisson mais éloigné de la cuve 22. L'appareil de cuisson est bien entendu associé à des moyens de chauffage, non représentés. Dans ce mode de réalisation, l'élément capacitif de mesure est suspendu, par des moyens d'accrochage 26 solidaires du substrat 8, à partir du rebord supérieur de la paroi latérale de la cuve 22 et s'étendant sensiblement parallèlement à cette paroi.

A la figure 3, on a représenté deux courbes C1, C 2 illustrant respectivement la variation de la capacité C de l'élément capacitif de mesure du dispositif 1 en fonction de la température T pour une même huile respectivement neuve et usagée. Par huile usagée on entend ici une huile ayant subie plusieurs cycles de cuisson. La courbe C1 correspond à la variation de la capacité de l'élément capacitif de mesure dans le cas où les électrodes 3 et 4 baignent dans une huile neuve, tandis que la courbe C2 correspond à la variation de la capacité de l'élément capacitif de mesure dans le cas où les électrodes 3 et 4 baignent dans une huile usagée. On constate que ces deux courbes évoluent généralement de la même manière en fonction de la température et notamment que pour une température donnée, la différence entre la capacité mesurée avec l'huile neuve et l'huile usagée est sensiblement constante. En conséquence, la mesure de la capacité de l'élément capacitif EFM permet aisément de discriminer une bonne huile d'une huile usagée dans une large gamme de température.

En se référant désormais à la figure 4, on voit un deuxième mode de réalisation d'un dispositif de mesure, dans lequel les éléments identiques à ceux déjà décrits sont désignés par les- mêmes références numériques. Ce dispositif sera également décrit dans le cadre d'une application à la mesure de la qualité et/ou de la dégradation d'une huile alimentaire F contenue dans la cuve 22 d'un appareil de cuisson 24.

Dans ce mode de réalisation, le capteur 2 comprend, en plus de l'élément capacitif de mesure EFM qui baigne dans l'huile à mesurer, un élément capacitif de référence EFR qui baigne dans une huile de référence F_{rèf.} disposée dans une enceinte pour être séparée de l'huile à mesurer. L'huile de référence est une huile ayant les mêmes caractéristiques que l'huile neuve à- mesurer. La structure de l'élément capacitif de référence EFR est de préférence identique à celle de l'élément capacitif de mesure EFM bien que cela ne soit pas indispensable. L'élément capacitif de référence EFR est donc formé des électrodes 4_{réf} et 6_{réf.} en combinaison avec l'huile de référence F_{réf.} L'élément capacitif de référence EFR est donc capable de fournir un signal de référence représentatif de la constante diélectrique de l'huile de référence, signal qui peut être comparé avec le signal de sortie de mesure provenant de l'élément capacitif de mesure EFM par des moyens de traitement 26. Le branchement de l'élément capacitif de mesure et de l'élément capacitif de référence aux moyens de traitement 26 est représenté schématiquement à la figure 4. Dans cet exemple, les moyens de traitement 26 comprennent typiquement un convertisseur capacité/tension 27 à trois entrées et une sortie tension analogique reliée à un microcontrôleur 28, qui est relié à son tour à des moyens d'affichage 30. Une première électrode 6, 4_{réf.} de chaque élément capacitif EFM et EFR est reliée à une première entrée commune du convertisseur tandis que les deuxièmes électrodes 4, 6_{réf}. de chaque élément capacitif EFM et EFR sont reliées respectivement à des deuxième et troisième entrées du convertisseur capacité/tension. A titre d'exemple, le circuit convertisseur capacité/tension 27 portant la référence XE2004 commercialisé par la société Xemics peut être utilisé et le microcontrôleur 28 est du même type que celui décrit en liaison avec le premier mode de réalisation.

Dans le mode de réalisation, illustré, l'élément capacitif de référence EFR et l'élément capacitif de mesure EFM sont disposés dans la cuve 22 de l'appareil de cuisson 24. L'élément capacitif de référence EFR est disposé dans une enceinte fermée 32 baignant dans l'huile à mesurer, l'enceinte 32 étant fermée de manière étanche de sorte que l'huile de référence contenue dans cette enceinte ne se mélange pas avec l'huile à mesurer contenue dans la cuve 24. L'élément capacitif de mesure EFM est quant à lui disposé dans une enceinte ajourée 34, par exemple dont les parois sont formées d'un grillage, de sorte qu'il baigne dans l'huile à mesurer. L'utilisation de telles parois forme un filtre qui protège les électrodes de l'élément capacitif de mesure EFM et permet notamment d'éviter que des particules solides en suspension dans l'huile viennent en contact avec celles-ci ce qui pourrait perturber la mesure. Bien entendu selon une variante de réalisation ces parois pourraient être omises.

On notera que les parois de l'enceinte 32 et les parois de l'enceinte ajourée 34 forment respectivement un cadre ou écran métallique de protection vis-à-vis de perturbations électriques externes et permet de réduire ainsi l'influence de ces perturbations au cours des mesures.

Pour des raisons de commodité, les enceintes 32 et 34 sont solidaires l'une de l'autre et forment une structure unitaire 36 qui est fixée à la cuve 22 de l'appareil de cuisson 24. De préférence et comme cela est illustré à la figure 4, les éléments capacitifs sont fixés dans leur enceinte respective par des supports isolants coopérant avec leur substrat.

On notera que l'enceinte 32 contenant l'huile de référence comprend un canal de remplissage 38, dont l'orifice peut être le cas échéant fermé de façon étanche par un bouchon ou un couvercle (non représenté). Selon une variante non représentée, l'enceinte 32 peut comprendre en outre des moyens de vidange disposés dans sa partie inférieure.

On notera également que la structure 36 est avantageusement disposée au voisinage d'une paroi verticale intérieure 22a de la cuve, ce qui permet de libérer une place suffisante pour cuire des aliments tout en effectuant des mesures.

La structure 36 comprend en outre sur une de ses parois latérales des moyens d'accrochage 40a, 40b destinés à coopérer avec des moyens d'accrochage complémentaires 42a, 42b solidaires de la paroi 22a. Dans l'exemple illustré, les moyens d'accrochage 40 et les moyens d'accrochage complémentaires 42 comprennent respectivement deux crochets 40a, 40b, 42a, et 42b coopérant deux à deux. La structure 36 peut ainsi être suspendue de façon amovible à l'intérieur de la cuve 22. On notera que ce montage amovible de la structure 36 facilite les opérations de remplissage et de vidage de l'enceinte 32 ainsi que les opérations d'entretien des éléments capacitifs EFM et EFR. Par ailleurs, comme la structure 36 est de construction simple et ne comporte en particulier aucune pièce mécanique mobile, elle présente une très grande fiabilité.

Dans ce mode de réalisation, les liaisons électriques entre les deux éléments capacitifs et les moyens de traitement 26 disposés à l'extérieur de la cuve se font via les crochets 40a, 40b, 42a, et 42b. A cet effet, les crochets 40a, 40b comprennent des plages de contact 44a, 44b reliées respectivement aux éléments capacitifs EFM et EFR. Les crochets 42a, et 42b comprennent des plages de contact complémentaires 46a, 46b reliées aux moyens de traitement 26 et destinées à venir en contact avec les plages de contact 44a, 44b lorsque la structure 26 est suspendue dans la cuve. La liaison électrique entre les plages de contact 44a, 44b et les éléments capacitifs est réalisée par des fils traversant de manière étanche la paroi de l'enceinte 32. De même, la liaison électrique entre les plages 46a, 46b et les moyens de traitement est réalisée par des fils traversant de manière étanche la paroi de la cuve 22. Bien entendu selon une variante de réalisation, les différentes connexions entre les éléments capacitifs et le circuit de traitement peuvent être indépendantes des moyens de suspension dans la cuve 22, être directement relié à un boîtier séparé pouvant ou non comprendre les moyens d'affichage 30 et formant avec la structure 36 une unité de mesure portable indépendante de tout appareil de cuisson.

A la figure 5 est représentée une variante du deuxième mode de réalisation du dispositif de mesure, dans laquelle le dispositif est associé à un système de renouvellement de l'huile de référence. Le système de renouvellement comprend un réservoir 48 contenant de l'huile neuve et disposé en dehors de la cuve 22. Le réservoir 48 est relié à un orifice d'entrée de l'enceinte 32 par une canalisation sur laquelle sont placées une pompe P et une électrovanne EV1. L'enceinte 32 est reliée par un orifice de sortie à une canalisation munie d'une électrovanne EV2 et débouchant dans la cuve 22. L'huile de référence usagée peut donc être ainsi réinjectée dans la cuve 22 et réutilisée.

On notera que la pompe P et les deux électrovannes EV1 et EV2 sont placées de préférence en dehors de la cuve 22 pour les protéger contre les effets de la température.

On notera encore que la pompe pourra être omise dans des variantes de réalisation dans lesquelles le réservoir 48 est placé suffisamment haut pour que l'huile à mesurer ne remonte pas dans l'enceinte contenant l'huile de référence lorsque les électrovannes sont ouvertes.

Ce système de renouvellement peut avantageusement être associé à des moyens pour commander de manière automatique et programmée la pompe et les électrovannes. Ces moyens peuvent typiquement prendre la forme d'un microprocesseur. Un tel système facilite l'exploitation du dispositif de mesure.

A figure 6 est représenté une variante du mode de réalisation illustré à la figure 5 dans laquelle le réservoir 48 est directement, relié à un orifice d'entrée de l'enceinte 32 par une canalisation 52. L'enceinte 32 est reliée par un orifice de sortie à une canalisation traversant un paroi de la cuve 22 et débouchant dans un réservoir de récupération 50. disposé à l'extérieur de la cuve 22. Le réservoir 48 est de préférence formé d'une poche étanche reliée à la canalisation 52 par un dispositif à écoulement contrôlé du type à goutte à goutte 54a et l'orifice de sortie de l'enceinte 32 est également associé à un dispositif à écoulement 54b contrôler du même type. Bien entendu les débits des deux dispositifs à écoulement contrôler sont identiques et peuvent avantageusement être réglé pour que le volume de l'enceinte 32 soit renouvelé quotidiennement. Un autre avantage de cette variante est que l'huile de référence est maintenue en tout temps à l'abri de l'oxygène.

L'avantage de cette variante réside dans le fait qu'elle ne comprend pas d'élément mécanique mobile, ce qui améliore sa fiabilité et facilite son entretien.

Avec le dispositif de mesure selon le deuxième mode de réalisation, l'élément capacitif de référence qui baigne dans une huile neuve sensiblement à la même température que l'huile à mesurer dans laquelle est disposé l'élément capacitif de mesure permet, en réalisant un montage en pont de wheatstone des deux éléments capacitifs, de distinguer les variations de la constante diélectrique de ces éléments, provenant de la dégradation de l'huile, des variations provenant des fluctuations de la température. Un tel montage en pont est décrit par exemple dans l'article intitulé « Application of capacitance techniques in sensor design » par Willem Chr. Heerens publié dans J. Phys. E : Scientific Instruments 19 :897 906 (1986), qui est incorporé dans cette demande par référence.

On comprendra que diverses modifications et/ou améliorations évidentes pour un homme du métier peuvent être apportées au mode de réalisation décrit dans la présente description sans sortir du cadre de la présente invention définie par les revendications annexées. En particulier on pourra envisager de disposer les éléments capacitifs une orientation quelconques dans la cuve contenant le fluide à mesurer.

On pourra également envisager de réaliser la structure unitaire 36 selon la configuration illustrée à la figure 7 dans laquelle les enceintes 32 et 34 sont agencées de manière plus compacte, les parois des enceintes ayant été omises pour laisser apparaître les éléments capacitifs EFM et EFR.

On pourra encore envisager dans le deuxième mode de réalisation d'agencer une électrode commune aux deux éléments capacitifs afin de limiter le nombre de connexions.

Il va de soi que le dispositif de mesure selon l'invention qui vient d'être décrit n'est pas limité à une application à la mesure d'huiles alimentaires et pourra être utilisé pour la mesure de la qualité et/ou de la dégradation de tout fluide dont l'évolution de sa constante diélectrique est représentatif de sa qualité et/ou de sa dégradation.

## Revendications

1. Utilisation d'un dispositif de mesure de la qualité et/ou de la dégradation d'un fluide pour la mesure de la qualité et/ou la dégradation d'une huile alimentaire, ledit dispositif comprenant :
un capteur destiné à être immergé dans ladite huile à mesurer, ledit capteur comportant au moins une paire d'électrodes espacées l'une de l'autre et s'étendant chacune sensiblement dans un même plan, chaque électrode de chaque paire d'électrodes présentant en outre la forme d'un peigne ayant une pluralité de dents sensiblement parallèles, les dents d'une des électrodes étant imbriquées entre les dents de l'autre électrode, les électrodes et ladite huile formant un élément capacitif de mesure dont la capacité varie en fonction de la constante diélectrique de ladite huile, ledit capteur étant capable de fournir un signal électrique de sortie représentatif de ladite constante diélectrique, et
des moyens de traitement recevant ledit signal de sortie et capables de déterminer le degré de qualité et/ou de dégradation de ladite huile sur la base dudit signal de sortie, utilisation dans laquelle ladite huile baigne les deux faces des électrodes, de part et d'autre dudit plan de sorte que ladite huile peut circuler en traversant ledit plan.

2. Utilisation selon la revendication 1 dudit dispositif de mesure, **caractérisée en ce que** le capteur comprend en outre un élément capacitif de référence comportant au moins une paire d'électrodes de référence espacées l'une de l'autre, ledit élément capacitif de référence étant destiné à être immergé dans une huile de référence, les électrodes et l'huile de référence formant un élément capacitif de référence dont la capacité varie en fonction de la constante diélectrique de l'huile de référence, ledit élément capacitif de référence étant capable de fournir un signal de référence représentatif de ladite constante diélectrique de référence audits moyens de traitement, et **en ce que** les moyens de traitement sont agencés pour comparer le signal de sortie avec le signal de référence.

3. Utilisation selon la revendication 2 dudit dispositif, **caractérisée en ce que** les électrodes dudit élément capacitif de référence s'étendent sensiblement dans un même plan et **en ce que** ladite huile de référence baigne les deux faces des électrodes dudit élément capacitif de référence, de part et d'autre leur plan de sorte que ladite huile peut circuler en traversant ledit plan.

4. Utilisation selon la revendication 2 ou 3 dudit dispositif, **caractérisée en ce que** le fluide de référence est disposé dans une enceinte isolée de ladite huile à mesurer et en contact thermique avec ce dernier, de sorte que ladite huile de référence présente sensiblement la même température que ladite huile à mesurer.

5. Utilisation selon la revendication 4 dudit dispositif, **caractérisée en ce que** l'enceinte contenant l'huile de référence est associée à un système de renouvellement de ladite huile de référence.

6. Utilisation selon la revendication 5 dudit dispositif, **caractérisée en ce que** ledit système de renouvellement comprend un réservoir d'huile de référence en communication avec ladite enceinte et **en ce que** ledit système comprend des moyens à écoulement contrôlé de manière à permettre un renouvellement régulier de l'huile de référence contenu dans ladite enceinte.

7. Utilisation selon l'une quelconque des revendications précédentes dudit dispositif, **caractérisée en ce que** les électrodes sont formées respectivement par des plaques planes.

8. Utilisation selon l'une quelconque des revendications précédentes dudit dispositif, **caractérisée en ce que** les éléments capacitifs sont entourées d'un cadre métallique formant écran contre les perturbations électromagnétiques.

9. Utilisation selon l'une quelconque des revendications précédentes dudit dispositif dans lequel les électrodes des éléments capacitifs sont réalisées à partir d'un acier alimentaire.

10. Utilisation selon l'une quelconque des revendications précédentes dudit dispositif, **caractérisée en ce que** les électrodes des éléments capacitifs sont portées par une structure de support électriquement isolante présentant une ouverture disposée en regard d'une région de mesure desdites électrodes.

11. Appareil de cuisson comprenant une cuve destinée à contenir un fluide de cuisson et des moyens de chauffage, **caractérisé en ce qu'**il comprend en outre un dispositif de mesure de la qualité et/ou de la dégradation dudit fluide de cuisson, ledit dispositif de mesure comprenant un capteur ayant au moins une paire d'électrodes espacées l'une de l'autre et s'étendant chacune sensiblement dans un même plan, chaque électrode de chaque paire d'électrodes présentant en outre la forme d'un peigne ayant une pluralité de dents sensiblement parallèles, les dents d'une des électrodes étant imbriquées entre les dents de l'autre électrode,'les électrodes et ledit fluide de cuisson formant un élément capacitif de mesure dont la capacité varie en fonction de la constante diélectrique de dudit fluide, ledit capteur étant capable de fournir un signal électrique de sortie représentatif de ladite constante diélectrique, et des moyens de traitement recevant ledit signal de sortie et capables de déterminer le degré de qualité et/ou de dégradation dudit fluide de cuisson sur la base dudit signal de sortie, l'élément capacitif de mesure étant disposé dans ladite cuve pour que ses électrodes baignent dans le fluide de cuisson par leur deux faces de part et d'autre dudit plan des électrodes de sorte que ledit fluide de cuisson peut circuler en traversant ledit plan,

12. Appareil de cuisson selon la revendication 11, **caractérisé en ce que** le capteur comprend en outre un élément capacitif de référence, comportant au moins une paire d'électrodes de référence espacées l'une de l'autre, destiné à être immergé dans un fluide, de référence, les électrodes et le fluide de référence formant un élément capacitif de référence dont la capacité varie en fonction de la constante diélectrique du fluide de référence, ledit élément capacitif de référence étant capable de fournir un signal de référence représentatif de ladite constante diélectrique de référence audits moyens de traitement, et **en ce que** les moyens de traitement sont agencés pour comparer le signal de sortie avec le signal de référence.

13. Appareil de cuisson selon la revendication 12, **caractérisé en ce que** les électrodes dudit élément capacitif de référence s'étendent sensiblement dans un même plan et **en ce que** le fluide de cuisson baigne les deux faces des électrodes de l'élément capacitif de référence, de part et d'autre dudit plan des électrodes de références.

14. Appareil de cuisson selon la revendication 12 ou 13, **caractérisé en ce que** le fluide de référence est disposé dans une enceinte isolée du fluide de cuisson à mesurer et en contact thermique avec ce dernier, de sorte que le fluide de référence présente sensiblement la même température que le fluide de cuisson à mesurer.

15. Appareil de cuisson selon la revendication 14, **caractérisé en ce que** l'enceinte contenant le fluide de référence est associée à un système de renouvellement dudit fluide de référence.

16. Appareil de cuisson selon la revendication 15, **caractérisé en ce que** ledit système de renouvellement comprend un réservoir de fluide de référence en communication avec ladite enceinte et **en ce que** ledit système comprend des moyens à écoulement contrôlé de manière à permettre un renouvellement régulier du fluide de référence contenu dans ladite enceinte.

17. Appareil de cuisson selon l'une quelconque des revendications 11 à 16, **caractérisé en ce que** les électrodes sont formées respectivement par des plaques planes.

18. Appareil de cuisson selon l'une quelconque des revendications 11 à 17, **caractérisé en ce que** les éléments capacitifs sont entourées d'un cadre métallique formant écran contre les perturbations électromagnétiques.

19. Appareil de cuisson selon l'une quelconque des revendications 11 à 18, **caractérisé en ce que** les électrodes des éléments capacitifs sont réalisées à partir d'un acier alimentaire.

20. Appareil de cuisson selon l'une quelconque des revendications 11 à 19, **caractérisé en ce que** les électrodes des éléments capacitifs sont portées par une structure de support électriquement isolante présentant une ouverture disposée en regard d'une région de mesure desdites électrodes.

## Claims

1. Use of a device for measuring the quality and/or degradation of a fluid, for measuring the quality and/or the degradation of a food oil, said device including:
a sensor to be immersed in said fluid to be measured, said sensor comprising at least one pair of electrodes spaced apart from each other and extending in substantially the same plane, each electrode of each pair of electrodes further having the shape of a comb having a having a plurality of substantially parallel teeth, the teeth of one of the electrodes being interdigited with the teeth of the other electrode, the electrodes and said oil forming a measuring capacitive element whose capacitance varies as a function of the dielectric constant of the oil, said sensor being capable of providing an electrical output signal representative of said dielectric constant, and
processing means receiving said output signal and capable of determining the degree of quality and/or degradation of said oil on the basis of said output signal,
wherein both sides of the electrodes are immersed in the fluid, on either side of said plane such that said oil can flow passing through said plane.

2. Use according to claim 1 of said measuring device, **characterized in that** the sensor further includes a reference capacitive element, comprising at least one pair of reference electrodes spaced apart from one another, said reference capacitive element being intended to be immersed in a reference oil, the electrodes and the reference fluid forming a reference capacitive element whose capacitance varies as a function of the dielectric constant of the reference oil, said reference capacitive element being capable of providing a reference signal representative of said reference dielectric constant to said processing means, and **in that** the processing means are arranged for comparing the output signal to the reference signal.

3. Use according to claim 2 of said device, **characterized in that** the electrodes of said reference capacitive element extend in substantially the same plane and **in that** both sides of the electrodes of said reference capacitive device are immersed in said oil, on either side of their plane, such that said oil can flow passing through said plane.

4. Use according to claim 2 or 3 of said device, **characterized in that** the reference fluid is arranged in an enclosed space insulated from said oil to be measured and in thermal contact with the latter, such that the reference oil has substantially the same temperature as said oil to be measured.

5. Use according to claim 4 of said device, **characterized in that** the enclosed space containing the reference oil is associated with a system for renewing said reference oil.

6. Use according to claim 5 of said device, **characterized in that** said renewal system comprises a reference oil tank in communication with said enclosed space and **in that** said system comprises flow control means so as to allow regular renewal of the reference oil contained in said enclosed space.

7. Use according to any one of the preceding claims of said device, **characterized in that** the electrodes are respectively formed by flat plates.

8. Use according to any one of the preceding claims of said device, **characterized in that** the capacitive elements are surrounded by a metal frame forming a screen against electromagnetic interference.

9. Use according to any one of the preceding claims of said, **characterized in that** the electrodes of the capacitive elements are made from a food grade steel.

10. Use according to any one of the preceding claims of said device, **characterized in that** the electrodes of the capacitive elements are carried by an electrically insulating support structure having an aperture opposite a measuring region of said electrodes.

11. Cooking apparatus including a vat for containing a cooking fluid and heating means, **characterized in that** it further includes a device for measuring the quality and/or degradation of said cooking fluid, said measuring device including a sensor having at least one pair of electrodes spaced apart from each other and extending in substantially the same plane, each electrode of each pair of electrodes further having the shape of a comb having a having a plurality of substantially parallel teeth, the teeth of one of the electrodes being interdigited with the teeth of the other electrode, the electrodes and said cooking fluid forming a measuring capacitive element whose capacitance varies as a function of the dielectric constant of the fluid, said sensor being capable of providing an electrical output signal representative of said dielectric constant, and processing means receiving said output signal and capable of determining the degree of quality and/or degradation of said cooking fluid on the basis of said output signal,
the measuring capacitive element being arranged in said vat such that both sides of its electrodes are immersed in the cooking fluid on either side of said plane of the electrodes so that said cooking fluid can flow passing through said plane.

12. Cooking apparatus according to claim 11, **characterized in that** the sensor further includes a reference capacitive element, including at least one pair of electrodes spaced apart from each other to be immersed in a reference fluid, the electrodes and the reference fluid forming a measuring capacitive element whose capacitance varies as a function of the dielectric constant of the reference fluid, said sensor being capable of providing an electrical output signal representative of said reference dielectric constant to said processing means and **in that** the processing means are arranged for comparing the output signal to the reference signal.

13. Cooking apparatus according to claim 12, **characterized in that** the electrodes of said reference capacitive element extend in substantially the same plane and **in that** the both sides of the reference capacitive element electrodes are immersed in said cooking fluid on either side of said plane of the reference electrodes.

14. Cooking apparatus according to claim 12 or 13, **characterized in that** the reference fluid is arranged in an enclosed space insulated from the cooking fluid to be measured and in thermal contact with the latter, such that the reference fluid has substantially the same temperature as the cooking fluid to be measured.

15. Cooking apparatus according to claim 14, **characterized in that** the enclosed space containing the reference fluid is associated with a system for renewing said reference fluid.

16. Cooking apparatus according to claim 15, **characterized in that** said renewal system includes a reference fluid tank in communication with said enclosed space and **in that** said system includes flow control means to allow regular renewal of the reference fluid contained in said enclosed space.

17. Cooking apparatus according to any one of claims 11 to 16, **characterized in that** the electrodes are respectively formed by flat plates.

18. Cooking apparatus according to any one of claims 11 to 17, **characterized in that** the capacitive elements are surrounded by a metal frame forming a screen against electromagnetic interference.

19. Cooking apparatus according to any one of claims 11 to 18, **characterized in that** the electrodes of the capacitive elements are made from a food grade steel.

20. Cooking apparatus according to any one of claims 11 to 19, **characterized in that** the electrodes of the capacitive elements are carried by an electrically insulating support structure having an aperture arranged facing a measuring region of said electrodes.

## Patentansprüche

1. Verwendung einer Vorrichtung zum Messen der Qualität und/oder der Verschlechterung eines Fluids, um die Qualität und/oder die Verschlechterung eines Speiseöls zu messen, wobei die Vorrichtung umfasst:
einen Sensor, der dazu bestimmt ist, in das zu messende Öl eingetaucht zu werden, wobei der Sensor wenigstens ein Paar voneinander beabstandeter Elektroden umfasst, die sich jeweils im Wesentlichen in derselben Ebene erstrecken, wobei jede Elektrode jedes Elektrodenpaars außerdem die Form eines Kamms aufweist, der mehrere im Wesentlichen parallele Zähne besitzt, wobei die Zähne einer der Elektroden mit den Zähnen der anderen Elektrode verschachtelt sind, wobei die Elektroden und das Öl ein kapazitives Messelement bilden, dessen Kapazität sich in Abhängigkeit von der Dielektrizitätskonstante des Öls ändert, wobei der Sensor ein elektrisches Ausgangssignal ausgeben kann, das die Dielektrizitätskonstante repräsentiert, und
Verarbeitungsmittel, die das Ausgangssignal empfangen und den Qualitätsgrad und/oder den Verschlechterungsgrad des Öls anhand des Ausgangssignals bestimmen können, wobei im Gebrauch das Öl die beiden Flächen der Elektroden beiderseits der Ebene umspült, derart, dass das Öl zirkulieren kann, indem es die Ebene durchquert.

2. Verwendung nach Anspruch 1 der Messvorrichtung, **dadurch gekennzeichnet, dass** der Sensor außerdem ein kapazitives Referenzelement umfasst, das wenigstens ein Paar voneinander beabstandeter Referenzelektroden besitzt, wobei das kapazitive Referenzelement dazu bestimmt ist, in ein Referenzöl eingetaucht zu werden, wobei die Referenzelektroden und das Referenzöl ein kapazitives Referenzelement bilden, dessen Kapazität sich in Abhängigkeit von der Dielektrizitätskonstante des Referenzöls ändert, wobei das kapazitive Referenzelement ein Referenzsignal, das die Referenz-Dielektrizitätskonstante repräsentiert, an die Verarbeitungsmittel liefern kann, und dass die Verarbeitungsmittel so beschaffen sind, dass sie das Ausgangssignal mit dem Referenzsignal vergleichen.

3. Verwendung nach Anspruch 2 der Vorrichtung, **dadurch gekennzeichnet, dass** sich die Elektroden des kapazitiven Referenzelements im Wesentlichen in derselben Ebene erstrecken und dass das Referenzöl die beiden Flächen der Elektroden des kapazitiven Referenzelements beiderseits ihrer Ebene umspült, derart, dass das Öl zirkulieren kann, indem es die Ebene durchquert.

4. Verwendung nach Anspruch 2 oder 3 der Vorrichtung, **dadurch gekennzeichnet, dass** das Referenzfluid in einem Behälter angeordnet ist, der von dem zu messenden Fluid isoliert und mit diesem in einem thermischen Kontakt ist, derart, dass das Referenzöl im Wesentlichen die gleiche Temperatur wie das zu messende Öl besitzt.

5. Verwendung nach Anspruch 4 der Vorrichtung, **dadurch gekennzeichnet, dass** der Behälter, der das Referenzöl enthält, einem System zum Erneuern des Referenzöls zugeordnet ist.

6. Verwendung nach Anspruch 5 der Vorrichtung, **dadurch gekennzeichnet, dass** das Erneuerungssystem einen Referenzöl-Vorratsbehälter umfasst, der mit dem Behälter in Verbindung steht, und dass das System Mittel mit gesteuerter Strömung umfasst, derart, dass eine regelmäßige Erneuerung des in dem Behälter enthaltenen Referenzöls möglich ist.

7. Verwendung nach einem der vorhergehenden Ansprüche der Vorrichtung, **dadurch gekennzeichnet, dass** die Elektroden jeweils durch ebene Platten gebildet sind.

8. Verwendung nach einem der vorhergehenden Ansprüche der Vorrichtung, **dadurch gekennzeichnet, dass** die kapazitiven Elemente von einem Metallrahmen umgeben sind, der eine Abschirmung vor elektromagnetischen Störungen bildet.

9. Verwendung nach einem der vorhergehenden Ansprüche der Vorrichtung, bei der die Elektroden der kapazitiven Elemente aus einem für Lebensmittel geeigneten Stahl verwirklicht sind.

10. Verwendung nach einem der vorhergehenden Ansprüche der Vorrichtung, **dadurch gekennzeichnet, dass** die Elektroden der kapazitiven Elemente durch eine elektrisch isolierende Trägerstruktur getragen werden, die eine Öffnung aufweist, die gegenüber einem Messbereich der Elektroden angeordnet ist.

11. Kochgerät, das ein Gefäß, das dazu bestimmt ist, ein Kochfluid aufzunehmen, und Heizmittel umfasst, **dadurch gekennzeichnet, dass** es außerdem eine Vorrichtung zum Messen der Qualität und/oder der Verschlechterung des Kochfluids umfasst, wobei die Messvorrichtung einen Sensor mit wenigstens einem Paar Elektroden, die voneinander beabstandet sind und sich jeweils in derselben Ebene erstrecken, umfasst, wobei jede Elektrode jedes Elektrodenpaars außerdem die Form eines Kamms besitzt, der mehrere im Wesentlichen parallele Zähne besitzt, wobei die Zähne einer der Elektroden mit den Zähnen der anderen Elektrode verschachtelt sind, wobei die Elektroden und das Kochfluid ein kapazitives Messelement bilden, dessen Kapazität sich in Abhängigkeit von der Dielektrizitätskonstante des Fluids ändert, wobei der Sensor ein elektrisches Ausgangssignal ausgeben kann, das die Dielektrizitätskonstante repräsentiert, und Verarbeitungsmittel umfasst, die das Ausgangssignal empfangen und den Qualitätsgrad und/oder den Verschlechterungsgrad des Kochfluids anhand des Ausgangssignals bestimmen können,
wobei das kapazitive Messelement in dem Gefäß angeordnet ist, damit seine Elektroden von dem Kochfluid an ihren beiden Flächen beiderseits der Ebene der Elektroden umspült werden, derart, dass das Kochfluid zirkulieren kann, indem es die Ebene durchquert.

12. Kochgerät nach Anspruch 11, **dadurch gekennzeichnet, dass** der Sensor außerdem ein kapazitives Referenzelement mit wenigstens einem Paar voneinander beabstandeter Referenzelektroden umfasst, das dazu bestimmt ist, in ein Referenzfluid eingetaucht zu werden, wobei die Elektroden und das Referenzfluid ein kapazitives Referenzelement bilden, dessen Kapazität sich in Abhängigkeit von der Dielektrizitätskonstante des Referenzfluids ändert, wobei das kapazitive Referenzelement ein Referenzsignal, das die Referenz-Dielektrizitätskonstante repräsentiert, an die Verarbeitungsmittel liefern kann und dass die Verarbeitungsmittel so beschaffen sind, dass sie das Ausgangssignal mit dem Referenzsignal vergleichen.

13. Kochgerät nach Anspruch 12, **dadurch gekennzeichnet, dass** sich die Elektroden des kapazitiven Referenzelements im Wesentlichen in derselben Ebene erstrecken und dass das Kochfluid die beiden Flächen der Elektroden des kapazitiven Referenzelements beiderseits der Ebene der Referenzelektroden umspült.

14. Kochgerät nach Anspruch 12 oder 13, **dadurch gekennzeichnet, dass** das Referenzfluid in einem Behälter angeordnet ist, der gegenüber dem zu messenden Kochfluid isoliert ist und mit diesem letzteren in einem thermischen Kontakt ist, derart, dass das Referenzfluid im Wesentlichen die gleiche Temperatur wie das zu messende Kochfluid besitzt.

15. Kochgerät nach Anspruch 14, **dadurch gekennzeichnet, dass** der das Referenzfluid enthaltende Behälter einem System zum Erneuern des Referenzfluids zugeordnet ist.

16. Kochgerät nach Anspruch 15, **dadurch gekennzeichnet, dass** das Erneuerungssystem einen Referenzfluid-Vorratsbehälter umfasst, der mit dem Behälter in Verbindung steht, und dass das System Mittel für eine gesteuerte Strömung umfasst, derart, dass eine regelmäßige Erneuerung des in dem Behälter enthaltenen Referenzfluids möglich ist.

17. Kochgerät nach einem der Ansprüche 11 bis 16, **dadurch gekennzeichnet, dass** die Elektroden jeweils durch ebene Platten gebildet sind.

18. Kochgerät nach einem der Ansprüche 11 bis 17, **dadurch gekennzeichnet, dass** die kapazitiven Elemente von einem Metallrahmen umgeben sind, der eine Abschirmung vor elektromagnetischen Störungen bildet.

19. Kochgerät nach einem der Ansprüche 11 bis 18, **dadurch gekennzeichnet, dass** die Elektroden der kapazitiven Elemente aus einem für Lebensmittel geeigneten Stahl verwirklicht sind.

20. Kochgerät nach einem der Ansprüche 11 bis 19, **dadurch gekennzeichnet, dass** die Elektroden der kapazitiven Elemente durch eine elektrisch isolierende Trägerstruktur getragen werden, die eine Öffnung aufweist, die gegenüber einem Messbereich der Elektroden angeordnet ist.
